# EUROPEAN PATENT SPECIFICATION

(11) **EP 1 872 710 B1**
(45) Date of publication and mention of the grant of the patent: **16.11.2011**
(21) Application number: 07111205.6
(22) Date of filing: 27.06.2007
(51) Int. Cl.: A61B 1/05, A61B 5/07

(54) **System and method for transmitting identification data in an in-vivo sensing device**
System und Verfahren zur Übertragung von Identifikationsdaten in einer In-vivo-Messvorrichtung
Système et procédé pour transmettre des données d'identification dans un dispositif de détection in vivo

(30) Priority: 30.06.2006 US 477743
(43) Date of publication of application: 02.01.2008
(73) Proprietor: Given Imaging Ltd., 20692 Yoqneam (IL)
(72) Inventor: Skala, Michael, 30900, Zichron Yaaqov (IL); Rubey, Kevin, Ventura, CA 93003 (US); Davidson, Tal, 20692, Yoqneam Illit (IL)
(74) Representative: Harrison Goddard Foote

(56) References cited:
- WO-A-97/33513
- DE-B3- 10 323 216
- US-A1- 2003 213 495
- US-A1- 2005 049 461

## Description

### FIELD OF THE INVENTION

The present invention relates to an apparatus and method for in-vivo imagine.

### BACKGROUND OF THE INVENTION

In-vivo devices, such as, for example, capsules, may be capable of gathering information regarding a body lumen while inside the body lumen. Such information may be, for example, a stream of data or image frames of the body lumen and/or measurements of sensed parameters, such as, for example, pH, temperature or other information. A sensing device may transmit sensed information via a hard-wired or wireless medium, and the information may be received by a receiver. The recorded information may be sent from the receiver to a workstation to be analyzed and/or displayed.

Such a system may be operated by, for example, health care professionals and technicians, in a hospital, or another health facility.

It is known from US 2003/0213495 to provide an in-vivo swallowable sensing capsule having a unique identification code stored in a non-volatile memory. The identification code is relatively long, and in order to reduce the amount of data transmitted by the capsule, an external reception system includes a management table in which the relatively long identification code is correlated with a relatively short capsule ID flag, and the short ID flag is then sent to the capsule and used in place of the relatively long identification code.

It is also known from WO 97/33513 to provide an implantable biosensing transponder that can be remotely interrogated by an appropriate system.

US 2005/0049461 discloses a capsule endoscope having an ID system for identifying horizontal/vertical lines of imaged data.

DE 103 23 216 discloses a swallowable capsule imaging device having two independently operable cameras, and tags Image files taken from the two cameras with ID codes so as to be able to distinguish between images taken with one camera and images taken with the other camera.

### SUMMARY OF THE INVENTION

According to the present invention, there is provided a method and system for in-vivo sensing as set forth in the appended claims.

The method and system for in-vivo sensing may transmit identification data that may relate to or identify the sensing device or a component within the device. The sensing device may transmit the identification data separately or together with sensory data, for example, in a data block. The identification data may be received, recorded, displayed, processed or used in any suitably way, for example, to verify, activate or select compatible in-vivo sensing system components.

### BRIEF DESCRIPTION OF THE DRAWINGS

Embodiments of the invention are illustrated by way of example and not limitation in the figures of the accompanying drawings, in which like reference numerals indicate corresponding, analogous or similar elements, and in which:
Fig. 1 is a simplified illustration of an in-vivo sensing system, including an in-vivo sensing device, a receiver and a workstation, in accordance with embodiments of the invention;
Fig. 2 is a schematic diagram of a block of data that may include identification data, in accordance with an embodiment of the invention; and
Fig. 3 is a flowchart of a method according to an embodiment of the present invention.

It will be appreciated that for simplicity and clarity of illustration, elements shown in the figures have not necessarily been drawn to scale. For example, the dimensions of some of the elements may be exaggerated relative to other elements for clarity.

### DETAILED DESCRIPTION OF THE INVENTION

In the following detailed description, numerous specific details are set forth in order to provide a thorough understanding of embodiments of the invention. However it will be understood by those of ordinary skill in the art that the embodiments of the invention may be practiced without these specific details. In other instances, well-known methods, procedures, components and circuits have not been described in detail so as not to obscure the embodiments of the invention.

Unless specifically stated otherwise, as apparent from the following discussions, it is appreciated that throughout the specification discussions utilizing terms such as "processing," "computing," "calculating," "determining," or the like, refer to the action and/or processes of a workstation, or similar electronic computing device, that manipulates and/or transforms data represented as physical, such as electronic, quantities within the computing system's registers and/or memories into other data similarly represented as physical quantities within the computing system's memories, registers or other such information storage, transmission or display devices.

An in-vivo sensing device may transmit identification data, which may include data that relates to or identifies the device, for example, device type, such as a model or brand, device components, supporting mechanisms, supporting software, compatibility requirements or other identifying data. Identification data may indicate what type of sensory data the sensing device collects, for example, image data, pH data, etc. Identification data may include areas in a patient's body where the sensing device may be used, for example, the colon, esophagus, etc. Identification data may include geographical zones or areas, for example, nations or geographical regions, where the sensing device and/or supporting system components may properly function, may be allowed to function, or may be compatible with other applications and/or systems. Identification data may include data that uniquely identifies the sensing device, for example, a code, serial number or electronic signature. In one embodiment, no two sensing devices may have precisely the same identification data. In other embodiments, a group or type of sensing devices may have the same identification data or a common portion of identification data.

The sensing device may also transmit sensory data, for example, image data, that the sensing device captures or collects while traversing a body. The sensing device may include an image sensor or camera, or components for sensing physiological parameters of a body lumen such as, pH, temperature, pressure, electrical impedance, etc.

Devices according to embodiments of the present invention may be similar to embodiments described in United States Patent Number 7,009,634 to Iddan et al., entitled "Device for In-Vivo Imaging", and/or in United States Patent Number 5,604,531 to Iddan et al., entitled "In-Vivo Video Camera System", and/or in United States Patent Application Number 10/046,541, filed on January 16, 2002, published on August 15, 2002 as United States Patent Application Publication Number 2002/0109774, all of which are hereby incorporated by reference. An external reception system or receiver unit, a processor and a monitor, e.g., in a workstation, such as those described in the above publications, may be suitable for use with some embodiments of the present invention. Devices and systems as described herein may have other configurations and/or other sets of components. For example, some embodiments of the present invention may be practiced using an endoscope, needle, stent, catheter, etc. Some in-vivo devices may be capsule shaped, or may have other shapes, for example, a peanut shape or tubular, spherical, conical, or other suitable shapes.

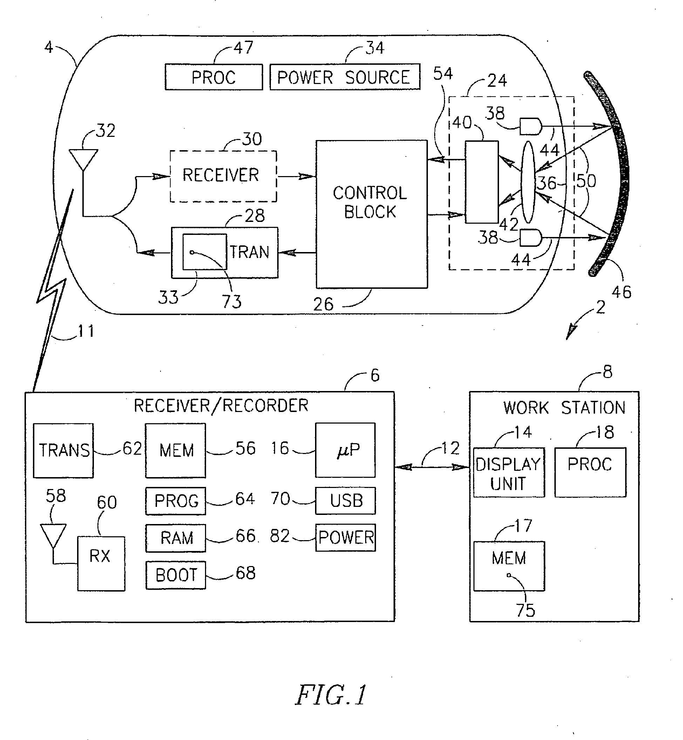

Reference is made to Fig. 1, which is a simplified illustration of an in-vivo sensing system 2, including an in-vivo sensing device 4, a receiver 6 and a processing system or workstation 8, in accordance with an embodiment of the invention. Receiver 6 may include a processor (uP) 16 to, for example, control, at least in part, the operation of receiver 6. Workstation 8 may include a processor 18, display unit 14 and memory 17 and may accept, process and/or display data received and/or recorded from receiver 6, which may include sensory data (e.g., image data) and/or identification data 73. In some embodiments receiver 6 separate from workstation 8 need not be used. Any unit which may receive or accept data transmitted by sensing device 4 may be considered a "reception system".

Sensing device 4 may include a control block 26, a transmitter 28, one or more memory units 33, a receiver 30, a processor 47, an antenna 32, a power source 34, and a sensing system 24. In one embodiment, sensing system 24 may include an imaging system that may include for example an optical window 36, at least one illumination source 38, such as, for example, a light emitting diode (LED), an imaging sensor 40, and an optical system 42. Sensing device 4 may include one or more registers or memory units 33, which may be included for example in processor 47, control block 26 or transmitter 28. In one embodiment, control block 26, processor 47 and transmitter 28, or all or part of their functionality may be combined in one unit. In one embodiment, components of sensing device 4 may be sealed within a device body, shell or container (the body, shell or container may include more than one piece).

According to one embodiment of the present invention, identification data 73 may be stored in the sensing device 4, for example, in memory unit 33, transmitter 28, processor 47, or any other storage area. In other embodiments identification data 73 may be stored using, for example, hard wired non-solid state devices, for example using one or more switches.

Transmitter 28 may transmit identification data 73. Identification data 73 may be transmitted automatically or in response to a system, program or administrator's request. Data, including for example sensory data and identification data 73, may be transmitted from the in-vivo sensing device 4 to receiver 6 via a wireless or hard-wired medium 11 while inside the patient's body. Receiver 6 may receive, record and/or store the data transmitted by transmitter 28. Receiver 6, which may be positioned close to or worn on a subject, may receive a stream of data transmitted by sensing device 4. Workstation 8 may download or access the stream of data from receiver 6 via, for example, a wireless or hard-wired medium 11, and may analyze and/or display the stream of data. In one embodiment, workstation 8 may download, store, use or display identification data 73 and sensory data, separately. In alternate embodiments workstation 8 may receive data transmitted directly from sensing device 4, rather than using receiver 6 as an intermediary.

In one embodiment, identification data 73 transmitted by sensing device 4, may be used to determine if sensing device 4 meets system 2 requirements, for example, identified by system 2 component's requirement data 75. Requirement data 75 may include, for example, data that specifies a system 2 component's requirement or standard, such that in order for the system 2 component to use sensory data transmitted by sensing devices 4, sensing devices 4 must transmit identification data 73 that substantially fulfills the requirement or standard. System 2 components, for example, receiver 6 and/or workstation 8, and applications and software thereof may include requirement data 75. In some embodiments, system 2 component requirement data 75 may be stored in the system 2 components themselves. For example, requirement data 75 may be stored in memory 17 of workstation 8 or memory 56 of receiver 6. Requirement data 75 may include, for example, read only data, electronic signatures or other types of data. Different system 2 components, as well as different hardware or software programs within a system 2 component, may have different identification requirements.

In one embodiment, a system 2 component may compare identification data 73 transmitted by the sensing device 4 with the requirement data 75. For example, sensing devices 4 must transmit identification data 73, which may be accepted by the system 2 component, for example, workstation 8, in order for system 2 components to work with sensing device 4. The system 2 component may read identification data 73. The system 2 component may read requirement data 75, which may be for example retrieved from memory. The system 2 component may compare analogous portions of identification data 73 and requirement data 75 to determine if the two data sets substantially match.

For example, workstation 8 may have requirement data 75 that specifies that workstation 8 may only use sensory data from sensing devices 4 that collect image data. Thus, if identification data 73 transmitted by sensing device 4 identifies sensing device 4 as an imaging device, workstations 8 may accept sensory data transmitted by sensing device 4.

In some embodiments, requirement data 75 may be entered at workstation 8, for example, by a user at a terminal. For example, a user may select a type of data or display program to be used by system 2, or configure workstation 8 or install software in workstation 8. For example, a user may configure workstation 8 by selecting a range of acceptable values, such that workstation 8 may only use sensory data from sensing devices 4 that transmit identification data 73 that falls within the range. In other embodiments, component requirement data 75 may include fixed or constant data, for example, pre-programmed, in hardware or software. In some embodiments, requirement data 75 or identification data 73 may be read-only data or may be protected or encrypted, such that the data may not be altered by a user.

In some embodiments, identification data 73 may include data indicating nations or geographical regions, in which sensing device 4 is intended to be used, function properly or comply with other system 2 components and applications. System 2 components may only accept or use sensory data from sensing device 4 if the regions in which sensing device 4 is intended to be used sufficiently matches region requirements of system 2 components. For example, a receiver 6 intended to be used in the United Kingdom may not receive, record and/or store sensory data transmitted by a sensing device 4 intended to be used in Australia.

In other embodiments, if identification data 73 includes data identifying the model, brand or type associated with sensing device 4, then system 2 components or applications may automatically access software such as programs, displays or modules that are compatible or preferably used with that model, brand or type of sensing device 4. For example, workstation 8 may accept identification data 73 including a model, version number, code or electronic signature, associated with sensing device 4, and may determine if identification data 73 matches requirement data 75 in the software. If identification data 73 sufficiently matches requirement data 75 in the software, workstation 8 may access or activate software or hardware that includes data that matches at least a portion of identification data 73. Thus, appropriate system 2 mechanisms may be accessed without instructions from a user. Receiver 6 and workstation 8 may accept identification data 73 and alter operations based on the identification data 73.

Identification data 73 may include data identifying the intended region in a patient's body from which sensing device 4 may collect sensory data, for example, the colon. Upon accepting such identification data 73, system 2 components or applications may access appropriate programs, displays, modules or software, for example, for viewing sensory data collected from that region. For example, system 2 may include localization tools or devices that may provide data on the location of sensing device 4 as it traverses the Gl tract. Workstation 8 may access a preferred localization display application for the intended region in the patient's body from which sensing device 4 collects sensory data. For example, workstation 8 may access a generic localization display program and superimpose a diagram, map or schematic illustration of the region, for example, the Gl tract, on a generic display.

In one embodiment, identification data 73 may include data that uniquely identifies sensing device 4, for example, a unique identifier such as a serial number, code or electronic signature. Multiple sensing devices 4 traversing one or more patients' bodies may transmit sensory data to receiver 6, for example, at overlapping times. Identification data 73 may be attached, grouped with or tagged onto the sensory data according to embodiments of the invention. Receiver 6 may separate the sensory data into separate image streams according to from which sensing device 4 the identification data 73 indicates the sensory data was transmitted. Thus, data collected from multiple sensing devices 4 at the same or overlapping time may be stored, used and displayed separately.

In some embodiments, additional identification data may be accepted at workstation 8, for example, that is entered or selected by a user. Such identification data may be used by system 2 components according to embodiments of the invention. In some embodiments, additional identification data may overwrite or replace transmitted identification data 73.

In some embodiments, identification data 73 transmitted by sensing device 4 may be stored, in a data structure or storage or memory location, with, or associated with sensory data transmitted by the same sensing device 4, for example, in receiver 6 or workstation 8, and be used for reference purposes. For example, identification data 73 may be used to identify sensing device 4 that collected the sensory data. In one embodiment, each frame of image data may include identification data. In another embodiment, each file of image data may include identification data. Other methods of associating identification data with sensory data may be used.

A non-exhaustive list of examples of processing system or workstation 8 includes a original equipment manufacturer (OEM) dedicated work station, a desktop personal computer, a server computer, a laptop computer, a notebook computer, a hand-held computer, and the like.

Receiver 6 may include a memory 56, for example, to store sensory and/or identification data transmitted from sensing device 4, a processor 16, an antenna 58, a receiver (RX), a transmitter 62, a program memory 64, a random access memory (RAM) 66, boot memory 68, a power source 82, and a communication controller, such as, for example, a universal serial bus (USB) controller 70. According to other embodiments of the invention, transmitter 62 may be a unit separate from receiver 6.

Processor 16 may control the operation of receiver 6, transmitter 62, and USB controller 70 through, for example, a bus 74. In addition, receiver 6, transmitter 62, processor 16 and USB controller 70 may exchange data, such as, for example, sensory data received from sensing device 4, or portions thereof, over bus 74. Other methods for control and data exchange are possible.

One or more antenna(s) 58 may be mounted inside or outside receiver 6 and both receiver 60 and transmitter 62 may be coupled to antenna 58. Transmitter 62 may transmit wireless messages to sensing device 4 through antenna 58. Receiver 6 may receive transmissions, for example, from sensing device 4 through antenna 58.

Receiver 6 may communicate with workstation 8 via connection or medium 12. For example, receiver 6 may transfer bits of wireless communication, for example, sensory data, identification data or other data stored in memory 56 to workstation 8, and may receive controls, and other digital content, from workstation 8. Although the invention is not limited in this respect, medium 12 may be, for example, a USB cable and may be coupled to USB controller 70 of receiver 6. Alternatively, medium 12 may be wireless, and receiver 6 and workstation 8 may communicate wirelessly.

A non-exhaustive list of examples of antennae 32 and 58 includes dipole antennae, monopole antennae, multilayer ceramic antennae, planar inverted-F antennae, loop antennae, shot antennae, dual antennae, omni-directional antennae, coil antennae or any other suitable antennas. Moreover, antenna 32 and antenna 58 may be of different types.
Sensing device 4 may be or may include an autonomous swallowable capsule, for example, an imaging capsule, but sensing device 4 may have other shapes and need not be swallowable or autonomous. Embodiments of sensing device 4 are typically autonomous, and are typically self-contained. For example, sensing device 4 may be a capsule or other unit where all the components including for example power components are substantially contained within a container or shell, and where sensing device 4 does not require any wires or cables to, for example, receive power or transmit information. Sensing device 4 may communicate with an external receiving and display system to provide display of data, control, or other functions. For example, in an autonomous system power may be provided by an internal battery or a wireless receiving system. Other embodiments may have other configurations and capabilities. For example, components may be distributed over multiple sites or units. Control information may be received from an external source.

A non-exhaustive list of examples of memory units 33 includes, for example, semiconductor devices such as registers, latches, electrically erasable programmable read only memory devices (EEPROM), flash memory devices, etc. At least one memory unit 33 may store identification data 73.

Power source 34 may include batteries, such as, for example, silver oxide batteries, lithium batteries, capacitors, or any other suitable power source. Power source 34 may receive power from an external power source, for example, by a magnetic field or electric field that transmits to the device.

Imaging sensor 40 may be for example a solid state imaging sensor or imager, a complementary metal oxide semiconductor (CMOS) imaging sensor, a charge coupled device (CCD) imaging sensor, a "camera on chip" imaging sensor, or any other suitable imaging sensor. A 256x256 or 320x320 pixel imager may be used. Pixel size may be, for example, between 5 and 6 micron. According to some embodiments pixels may be each fitted with a micro lens. Other numbers or dimensions may be used.

Control block 26 may control, at least in part, the operation of sensing device 4. For example, control block 26 may synchronize time periods, in which illumination source 38 produce light rays, time periods, in which imaging sensor 40 captures images, and time periods, in which transmitter 28 transmits the images. In addition, control block 26 may produce timing signals and other signals necessary for the operation of transmitter 28, receiver 30 and imaging sensor 40. Moreover, control block 26 may perform operations that are complimentary to the operations performed by other components of sensing device 4, such as, for example, image data buffering. Identification data 73 may be used to control the mode or setting for control block 26, processor 47 or image sensor 40. Control block 26 may include any combination of logic components, such as, for example, combinatorial logic, state machines, controllers, processors, memory elements, and the like.

Control block 26, transmitter 28, optional receiver 30 and imaging sensor 40 may be implemented on any suitable combination of semiconductor dies or chips. For example, and although the invention is not limited in this respect, control block 26, transmitter 28 and optional receiver 30 may be parts of a first semiconductor die or chip, and imaging sensor 40 may be a part of a second semiconductor die. Such a semiconductor die may be an application-specific integrated circuit (ASIC) or may be part of an application-specific standard product (ASSP). According to some embodiments semiconductor dies may be stacked. According to some embodiments some or all of the components may be on the same semiconductor die.

Illumination source 38 may produce light rays 44 that may penetrate through optical window 36 and may illuminate an inner portion 46 of a body lumen. A non-exhaustive list of examples of body lumens includes the gastrointestinal (GI) tract, a blood vessel, a reproductive tract, or any other suitable body lumen.

Reflections 50 of light rays 44 from inner portion 46 of a body lumen may penetrate optical window 36 back into sensing device 4 and may be focused by optical system 42 onto imaging sensor 40. Imaging sensor 40 may receive the focused reflections 50, and in response to an image capturing command from control block 26, imaging sensor 40 may capture image data or an image of inner portion 46 of a body lumen. Control block 26 may receive the image of inner portion 46 from imaging sensor 40 over wires 54, and may control transmitter 28 to transmit the image of inner portion 46 through antenna 32 into wireless medium 11. Optional processor 47 may modify control block 26 operations.

Sensing device 4 may passively or actively progress along a body lumen. Consequently, a stream of sensory data of inner portions of a body lumen may be transmitted from sensing device 4 into wireless medium 11.

Sensing device 4 may transmit captured images embedded in, for example, "wireless communication frames". A payload portion of a wireless communication frame may include a captured image or other sensing data and may include additional data, such as, for example, identification data 73, telemetry information and/or cyclic redundancy code (CRC) and/or error correction code (ECC). In addition, a wireless communication frame may include an overhead portion that may contain, for example, framing bits, synchronization bits, preamble bits, and the like. Identification data 73 may be sent separately from image frames.

Receiver 30 may receive wireless messages via wireless medium 11 through antenna 32, and control block 26 may capture these messages. A non-exhaustive list of examples of such messages includes modifying the operations of sensing device 4, for example, activating or de-activating image capturing by sensing device 4 and/or activating or de-activating transmissions from sensing device 4, based on transmitted identification data 73.
Typically, the sensing device transmits data that are fixed in size. Typically, the sensing device collects data at a constant rate. For example, sensing device 4 may capture an image once every half second, and, after capturing such an image data may be transmitted the image to receiver 6 as an encoded image possibly over a series of imaging and transmission periods. A transmission or imaging period may be a period of time during which the sensing device may collect, generate and/or transmit a stream of sensory data. For example, in each of a series of transmission periods, a frame of image data may be captured and transmitted. Other constant and/or variable capture rates and/or transmission rates may be used. Typically, the image data recorded and transmitted is digital color image data, although in alternate embodiments other image formats (e.g., black and white image data) may be used. In one embodiment, each frame of image data may include, for example, 256 rows of 256 pixels or 320 rows of 320 pixels each, each pixel including data for color and brightness, according to known methods. Other data formats may be used.

In one embodiment, identification data 73 may be transmitted once at the start and/or once at the end of the collection and/or transmission of sensory data from sensing device 4. In such embodiments, identification data 73 may be used to indicate or command the start or end of data transmissions from sensing devices 4. For example, after receiver 6 receives identification data 73, indicating the completion of the transmission or reception of image data corresponding to an image frame. Upon receiving such indications, receiver 6 may deactivate receiving operations. In another embodiment, identification data 73 may be transmitted once at the start and/or once at the end of the movement of sensing device 4 across a region of a patient's body. Such markers may be used by receiver 6 and/or workstation 8 to sort or group sensory data (e.g., by image or frame).

The location of identification data 73 in transmitted data streams may be fixed or otherwise indicated, for example, by a data marker, pointer or an address, which may be easily accessible to a user or program applications. This may enable receiver 6, workstation 8 or a user to efficiently locate and access identification data 73.

In one embodiment, sensing device 4 may transmit identification data 73 separately from sensory data. For example, if sensory data corresponding to an image frame is not transmitted (e.g. due to functional error) identification data 73 corresponding to the image frame may still be transmitted.

In another embodiment, sensing device 4 may transmit identification data 73 together with sensory data, for example, in substantially the same data block, data stream or transmission or imaging period. Identification data 73 may be transmitted with sensory data, for example, with every or substantially every data transmission, image frame transmission or during substantially every transmission or imaging period. In one embodiment, receiving identification data may indicate the completion of the transmission of image data corresponding to an image frame. In some embodiments, relatively low data transmission rates may be used, for example, in accordance with regulations. Transmitting identification data 73 with substantially every image data transmission may enable receiver 6 and/or workstation 8 to access the identification data 73 without requesting it from sensing device 4, which may be temporally inefficient or may take time, where time constraints may be an issue. In another embodiment, identification data 73 may be transmitted less often than sensory data.

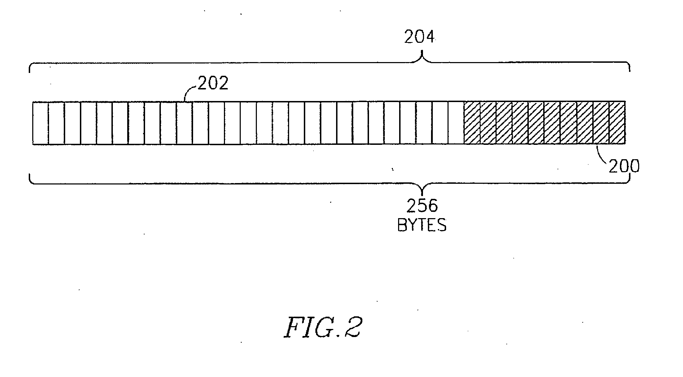

Reference is made to Fig. 2, a schematic diagram of a data block that may include identification data in accordance with an exemplary embodiment of the present invention. In some embodiments, sensing device 4 may transmit data in groups or blocks, for example, data block 204. Data block 204 may include sub-block 200 and sub-block 202. Sub-block 202 may include sensory data and sub-block 200 may include additional data such as identification data 73. In Fig. 2 sub-block 200 is located at the end of data block 204 for illustrative purposes, however, bytes including identification data 73 may be located in other locations within data block 204. For example, identification data 73 may be located at the beginning of data block 204.

In one embodiment sub-block 200 and sub-block 202 may package data in lines, sets, items or units of data that are typically a fixed size. For example, sub-block 202 may include a fixed number of bytes corresponding to the, for example, 256x262 pixels or 320x320 pixels of an image frame. In one embodiment, sensory data corresponding to each pixel in the image frame may have a fixed size, for example, 8 bits or 12 bits. Other block sizes or data formats may be used. Data block 204 may be any suitable length or size. While the length or size of data block 204 is typically fixed across transmission periods, the length may vary in some embodiments and/or transmissions.

Sub-block 200 may store multiple types of identification data 73. In one embodiment, specific types of identification data 73 may be grouped or transmitted in specific segments of sub-block 200, for example, in portions of in sub-block 200 that are fixed in size and position. Thus, system 2 components may automatically or efficiently access a desired specific type of identification data 73. For example, the unique identifier, geographical region data, body region data and model data may be transmitted in portions 250, 260, 270 and 280 of sub-block 200, respectively. Portions 250, 260, 270 and 280 of sub-block 200 may be arranged in any order in sub-block 200. Other data may be transmitted adjacent to or in between portions 250, 260, 270 and 280 of sub-block 200. Data block 204 may include a marker or address that identifies the location of identification data 73 in data block 204.

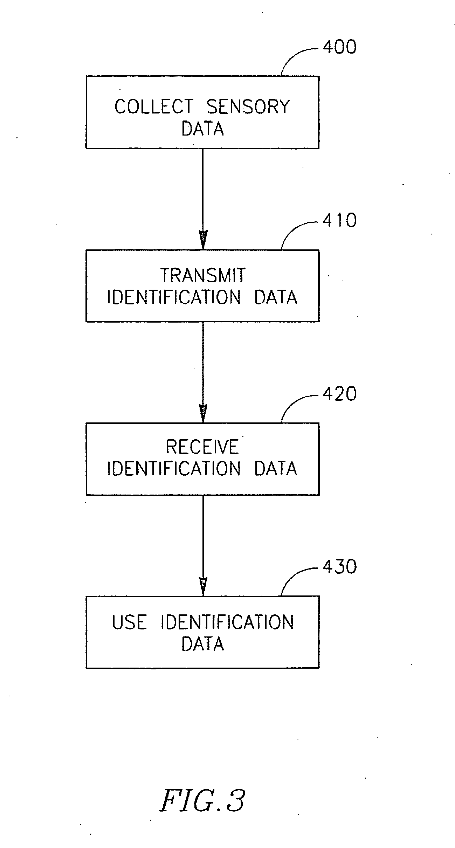

Fig. 3 is a flowchart of a method according to an embodiment of the present invention.

In operation 400, an in-vivo sensing device may collect sensory data. Sensory data may include, for example, image data collected or captured using an imaging system. For example, an autonomous in-vivo imaging device may capture image data. In other embodiments, sensory data may include, for example, data relating to pH, temperature, pressure, electrical impedance, or other sensed information.

In operation 410, identification data may be transmitted. The identification data may be transmitted alone or with the sensory data. Identification data may be attached to or grouped, packaged, transmitted or associated with sensory data, for example, in a data block or transmission period. In one embodiment, during one transmission period, data may be transmitted that includes image data and identification data.

In operation 420, a receiver may receive identification data, and may record or store the identification data. The receiver may send the identification data to a processing system such as a workstation via a wireless or hard-wired medium. The identification data may be sent alone or with the sensory data.

In operation 430, an in-vivo sensing system may use the identification data. For example, the workstation and/or receiver may store, process, display or use the sensory data in a suitable manner, for example, as allowed by the identification data. For example, identification data may be used to verify component compatibility or permissions, to allow access, or to select compatible system software or components, preferred operation settings, programs or software. System operation may be modified according to for the identification data. Identification data may have other meaning or functionality.

In one embodiment, a system component may compare the identification data transmitted by the sensing device with the system component's requirement data. For example, the system component may compare analogous portions of the identification data and requirement data to determine if the two data sets substantially match. In some embodiments, the system component may only use sensory data transmitted by the sensing devices if the sensing devices transmits identification data that matches the requirement data.

Other operations or series of operations may be used.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

It will be appreciated by persons skilled in the art that the present invention is not limited to what has been particularly shown and described hereinabove. Rather the scope of the present invention is defined only by the claims, which follow:

## Claims

1. A method for in-vivo sensing comprising:
capturing sensory data in an in-vivo swallowable sensing device (4);
transmitting, from said in-vivo swallowable sensing device (4), sensory data and identification data (73);
receiving, at a reception system (6,8), the identification data (73) from said in-vivo swallowable sensing device (4);
**characterized in that**:
the received identification data (73) includes location data indicating an area of the body where the sensing device (4) is intended to be used, and **in that**, based on said location data, an appropriate display application for viewing the sensory data collected from the intended area of the body is activated.

2. The method of claim 1, wherein the identification data (73) further includes data identifying a model, brand or type associated with the in-vivo swallowable sensing device and optionally data identifying device components, supporting mechanisms, supporting software and/or compatibility requirements.

3. The method of claim 1 or 2, wherein the identification data (73) further includes data indicating a geographical region and/or a unique identifier for the device and/or a version number associated with the device and/or an electronic signature.

4. The method of any preceding claim, comprising transmitting the sensory data and the identification data (73) during one transmission period and/or as a data block (204).

5. The method of any preceding claim, wherein transmitting the identification data (73) indicates the completion of the transmission of sensory data corresponding to an image frame.

6. An in-vivo sensing system (2) comprising:
an in-vivo swallowable sensing capsule (4), the sensing capsule (4) transmitting sensory data and identification data (73); and
a reception system (6,8) to receive the identification data (73) from said in-vivo swallowable sensing capsule (4);
**characterized in that**:
the received identification data (73) includes location data indicating an intended area of the body where the sensing capsule (4) is intended to be used and **in that** the reception system (6,8) is configured to activate, based on said location data, an appropriate display application for viewing the sensory data collected from the intended area of the body.

7. The system of claim 6, wherein the reception system (6,8) comprises a receiver (6) and a separate workstation (8) coupled to the receiver (6).

8. The system of claim 6, wherein the reception system (6,8) comprises a workstation (8) with an integral receiver (6).

9. The system of any one of claims 6 to 8, wherein the identification data (73) further includes data identifying a model, brand or type associated with the in-vivo swallowable sensing capsule (4) and optionally data identifying device components, supporting mechanisms, supporting software and/or compatibility requirements.

10. The system of any one of claims 6 to 9, wherein the identification data (73) further includes data indicating a geographical region and/or a unique identifier for the capsule and/or a version number associated with the capsule and/or an electronic signature.

11. The system of any one of claims 6 to 10, wherein the reception system (6,8) includes or is linked to a processing system (16,17,18,56) that performs the activation of the appropriate display application.

12. The system of claim 11, wherein the processing system (16,17,18,56) downloads the sensing information and the identification information (73) from the reception system (6,8).

13. The system of claim 11 or 12, wherein the processing system (16,17,18,56) is to activate software based on the identification data (73).

## Patentansprüche

1. Verfahren zur tn-vivo-Messwerterfassung, umfassend:
Erfassen sensorischer Daten in in-vivo schluckbaren ensorvorrichtung (4);
Übertragung von sensorischen Daten und Identifikationsdaten (73) von der in-vivo schluckbaren Sennsorvorrichtung (4);
Empfang der Identifikationsdaten (73) aus der genannten in-vivo schluckbaren Sensorvorrichtung (4) an ein Empfangssystem (6,8);
**dadurch gekennzeichnet, dass**
die empfangenen Identifikationsdaten (73) Ortungsdaten einschlieen, welche ein Gebiet des Körpers umfassen, in dem die Verwendung der Sensorvorrichtung (4) ist, und basierend auf den genannten Ortungsdaten eine geeignete Anzeige-Applikation zur Betrachtung der aus dem beabsichtigten Körpergebiet gesammelten sensorischen Daten aktiviert wird.

2. Verfahren nach Anspruch 1, die Identifikationsdaten (73) weiterhin Daten umfassen, eich in Modell, ein Marke oder einen Typ identifizieren, das oder die oder der der in-vivo schluckbaren Sensorvorrichtung (4) zugeordnet ist, und optional Daten umfassen, welche Vorrichtungskomponenten, n, Unterstützungsmechanismen, Unterstützungssoftware und/oder Kompatibilitätserfordernisse identifizieren.

3. Verfahren nach Anspruch 1 oder 2, die Identifikationsdaten (73) weiterhin Daten umfassen, welche eine geographische Region und/oder eine eindeutige Kennung für die Vorrichtung und/oder eine der Vorrichtung zugeordnete Versionsnummer und/oder eine elektronische Signatur identifizieren.

4. Verfahren nach einem der vorhergehenden Ansprüche, umfassend die Übertragung der sensorischen Daten und der Identifikationsdaten (73) während einer Übertragungsperiode und/oder als ein Datenblock (204).

5. Verfahren nach einem der vorhergehenden Ansprüche, wobei zur die Übertragung der (73) den schluss der Übertragung der sensorischen Daten, die einem Einzelbild entsprechen, anzeigt.

6. In-vivo-Erfassungssystem (2) mit:
einer vivo schluckbaren Sensorkapsel (4), wobei die Sensorkapsel (4) sensorische Daten und Identifikationsdaten (73) überträgt; und
einem Empfangssystem (6,8) zum Empfang der Identifikationsdaten (73) von der genannten in-vivo schluckbaren Sensorkapsel (4);
**dadurch gekennzeichnet, dass**
die empfangenen Identifikationsdaten (73) Ortungsdaten einschlieβen, welche ein des Körpers umfassen, in dem die Verwendung der Sensorvorrichtung (4) beabsichtigt ist, und dass das Empfangssystem (6,8) dazu eingerichtet ist, basierend auf den genannten Ortungsdaten eine geeignete Anzeige-Applikation zur Betrachtung der aus dem beabsichtigten Körpergebiet gesammelten sensorischen Daten zu aktivieren.

7. System nach Anspruch 6, wobei das Empfangssystem (6,8) einen Empfänger (6) und ein separates mit dem Empfänger (6) gekoppelten Arbeitsplatzsystem (8) aufweist.

8. System nach Anspruch 6, wobei das Empfangssystem (6,8) ein Arbeitsplatzsystem (8) mit integriertem Empfänger (6) aufweist.

9. System nach einem der Ansprüche 6 bis 8, wobei die Identifikationsdaten (73) weiterhin Daten umfassen, welche ein Modell, eine Marke oder einen Typ identifizieren, das oder die oder der der in-vivo schluckbaren Sensorvorrichtung (4) zugeordnet ist, und optional Daten umfassen, welche Vorrichtungskomponenten, Unterstützung chanismen, Unterstützungssoftware und/oder Kompatibilitätsedordernisse identifizieren.

10. System nach in Ansprüche 6 bis 9, wobei die Identifikationsdaten (73) weiterhin Daten umfassen, welche eine geographische Region und/oder eine eindeutige Kennung für die Kapsel und/oder eine der Kapsel zugeordnete Versionsnummer und/oder eine elektronische Signatur identifizieren.

11. System nach eine der Ansprüche 6 bis 10, das Empfangssystem (6,8) ein Verarbeitungssystem (16,17,18,56) enthält oder diesem zugeordnet ist, die der geeigneten Anzeige-Applikation ausführt.

12. System nach Anspruch 11, wobei das Verarbeitungssystem (16,17,18,56) die und die Identifikationsinformationen (73) von dem Empfangssystem (6,8) herunterlädt.

13. System nach Anspruch 11 oder 12, wobei das Verarbeitungssystem (16,17,18,56) Software auf der der Identifikationsdaten (73) aktiviert.

## Revendications

1. Procédé pour la détection in vivo comprenant les étapes de :
- saisir des données sensorielles dans un dispositif de détection (4) susceptible d'être avalé in vivo.
- transmettre, à partir dudit dispositif (4) de détection in vivo susceptible d'être avalé, les données sensorielles et les données d'identification (73) ;
- recevoir, en un système de réception (6, 8) les données d'indentification (73) depuis ledit dispositif (4) de détection in vivo susceptible d'être avalé;
**caractérisé en ce que** les données d'identification reçues incluent les données de localisation indiquant une zone du corps où le dispositif de détection (4) est destiné à être utilisé, et **en ce que**, sur la base de ladite donnée de localisation, une application d'affichage appropriée pour visualiser les données sensorielles collectées à partir de la zone envisagée du corps est activée.

2. Procédé selon la revendication 1, **caractérisé en ce que** les données d'identification (73) incluent en outre des données d'identification d'un modèle, type ou marque, associées au dispositif de détection in vivo susceptible d'être avalé, et optionnellement des données identifiant les composants de dispositif, supportant des mécanismes, supportant des logiciels et/ou des éléments nécessaires pour la compatibilité.

3. Procédé selon la revendication 1 ou 2, **caractérisé en ce que** les données d'identification (73) incluent en outre des données indiquant une région géographique et/ou un identifiant unique pour le dispositif et/ou un numéro de version associé au dispositif et/ou à une signature électronique.

4. Procédé selon l'une des revendications précédentes, **caractérisé en ce qu'**il comporte l'étape de transmettre les données sensorielles et les données d'identification (73) durant une période de transmission et/ou en tant que bloc de données (204).

5. Procédé selon l'une des revendications précédentes, **caractérisé en ce que** la transmission des données d'identification (73) indique la complétion de la transmission des données sensorielles correspondant à un cadre d'image.

6. Système de détection in vivo (2) comprenant :
- une capsule (4) de détection in vivo susceptible d'être avalée, la capsule de détection (4) transmettant des données sensorielles et des données d'identification (73) ; et
- un système de réception (6, 8) pour recevoir les données d'identification (73) à partir de la capsule (4) de détection in vivo susceptible d'être avalée, **caractérisé en ce que** les données d'identification reçues (73) incluent des données de localisation indiquant une zone envisagée du corps où la capsule (4) est destinée à être utilisée et **en ce que** le système de réception (6, 8) est configuré pour activer, sur la base de ladite donnée de localisation, une application d'affichage appropriée pour visualiser les données sensorielles recueillies depuis la zone envisagée du corps.

7. Système selon la revendication 6, **caractérisé en ce que** le système de réception (6, 8) comporte un récepteur (6) et une station de travail séparée (8), couplée au récepteur (6).

8. Système selon la revendication 6, **caractérisé en ce que** le système de réception (6, 8) comporte une station de travail (8) avec un récepteur intégré (6).

9. Système selon l'une des revendication 6 à 8, **caractérisé en ce que** les données d'identification (73) incluent en outre des données identifiant un modèle, marque ou type associés à la capsule (4) de détection in vivo susceptible d'être avalée, et optionnellement des données d'identification des composants de dispositif, supportant des mécanismes, supportant des logiciels et/ou des dispositifs nécessaires à la compatibilité.

10. Système selon l'une des revendication 6 à 9, **caractérisé en ce que** les données d'identification (73) comportent en outre des données indiquant une région géographique et/ou un identifiant unique pour la capsule et/ou un nombre de version associée à la capsule et/ou à une signature électronique.

11. Système selon l'une des revendications 6 à 10, **caractérisé en ce que** le système de réception (6,8) inclut ou est lié à un système de traitement (16, 17, 18, 56) qui réalise l'activation de l'application d'affichage appropriée.

12. Système selon la revendication 11, **caractérisé en ce que** le système de traitement (16, 17, 18, 56) charge l'information de détection et l'information d'identification (73) à partir du système de réception (6, 8).

13. Système selon l'une des revendications 11 ou 12, **caractérisé en ce que** le système de traitement (16, 17, 18, 56) est apte à activer un logiciel basé sur les données d'identification (73).
